Europäisches Patentamt

(19) European Patent Office

Office européen des brevets

(11) Veröffentlichungsnummer: **0 002 255**
**B1**

(12) EUROPÄISCHE PATENTSCHRIFT

(45) Veröffentlichungstag der Patentschrift:
27.01.82

(21) Anmeldenummer: **78101484.0**

(22) Anmeldetag: **30.11.78**

(51) Int. Cl.³: **G 01 D 15/28** // A61B5/04

(54) **Registriergerät.**

(30) Priorität: **05.12.77 DE 2754108**

(43) Veröffentlichungstag der Anmeldung:
**13.06.79 Patentblatt 79/12**

(45) Bekanntmachung des Hinweises auf die Patenterteilung:
**27.01.82 Patentblatt 82/4**

(84) Benannte Vertragsstaaten:
**BE CH DE FR GB NL SE**

(56) Entgegenhaltungen:
**CH-A-322 678**
**DE-B-1 167 048**
**GB-A-634 575**
**GB-845 523**
**US-A-3 082 970**

(73) Patentinhaber: **SIEMENS AKTIENGESELLSCHAFT Berlin
und München, Postfach 22 02 61, D-8000 München 22 (DE)**

(72) Erfinder: **Ebert, Hans, Hedenusstrasse 1,
D-8520 Erlangen (DE)**

BUNDESDRUCKEREI BERLIN

Registriergerät

Die Erfindung bezieht sich auf ein Registriergerät zum Aufzeichnen elektrischer Signale auf einen bewegten bandförmigen Aufzeichnungsträger, mit einem Gerätegehäuse, das einen Antrieb mit Transportrolle für den Aufzeichnungsträger aufweist, und mit einem separaten, in das Gehäuse einsetzbaren Trägerschlitten als Träger eines Vorrates des Aufzeichnungsträgers, wobei der Trägerschlitten eine Schreibkante für wenigstens einen Schreibzeiger aufweist und Trägerschlitten und/oder Gerätegehäuse Andruck- bzw. Umlenkrollen für den Transport des Aufzeichnungsträgers umfassen, von denen eine Andruckrolle im Trägerschlitten gegen den Druck einer Feder verschiebbar gelagert ist, und wobei der Trägerschlitten schreibkantenseitig ein Formteil etwa in Form der im Betriebszustand gebildeten Spur des Aufzeichnungsträgers bildet, längs dessen der Aufzeichnungsträger lose anlegbar ist und das beim Einsetzen des Trägerschlittens den Aufzeichnungsträger mittels der Andruckrolle entgegen der Wirkung der Feder an die Transportrolle andrückt.

Besonders für tragbare Kardiographen ist es wichtig, das Registriergerät zum Aufzeichnen der vom Patienten abgenommenen physiologischen Signale einfach und funktionssicher aufzubauen. Dabei sollen einerseits gute Aufzeichnungseigenschaften und andererseits gute Bedienungseigenschaften gewährleistet sein. Befriedigende Aufzeichnungseigenschaften werden im wesentlichen durch eine spezielle Geometrie von Andruck- und Umlenkrollen sowie Schreibkante bestimmt, wobei im allgemeinen dadurch ein kompliziertes Einfädeln des Aufzeichnungsträgers notwendig wird. Insbesondere die Bedienung der Kardiographen soll aber bei Austausch und Neubestückung mit Aufzeichnungsträgern schnell und problemlos ermöglicht werden. Dies ist auch deswegen von besonderer Wichtigkeit, da im Routinebetrieb solche Geräte von technisch nicht vorgebildeten Personen bedient werden. Aus der CH-A-322 678 ist bereits ein derartiges Registriergerät bekannt, bei dem zum Ersatz des Aufzeichnungsträgers ein separater Trägerschlitten aus dem Gerätegehäuse herausnehmbar und nach Bestückung mit dem Aufzeichnungsträger mittels Führungsstifte in schlitzartige Führungen des Gerätegehäuses einhängbar ist. Eine gegen den Druck einer Feder verschiebbare Rolle gewährleistet dabei die Arretierung des Trägerschlittens. Weiterhin ist aus der US-A 3 082 970 ein Registriergerät vorbekannt, das einen im Gehäuse verschwenkbaren Trägerschlitten mit einem Vorformteil aufweist. Bei ausgeschwenktem Trägerschlitten wird dabei die Vorratsrolle am Aufzeichnungsträger ausgetauscht, das vordere Ende mit der einen Hand um das Formteil gelegt und mit der anderen Hand der gesamte Schlitten wieder in die Betriebsstellung geschwenkt. Bei solcher Handhabung muß besonders darauf geachtet werden, daß der Aufzeichnungsträger in die exakte Position gelangt und stramm zwischen den Rollen geführt ist, um hinreichend gute Aufzeichnungseigenschaften zu gewährleisten. Gleiches gilt im wesentlichen auch für das aus der GB-A-845 523 vorbekannte Registriergerät. Allen diesen bekannten Geräten ist dabei gemeinsam, daß der Umschlingungswinkel der Schreibkante durch den Aufzeichnungsträger kleiner oder höchstens 180° beträgt, was aber keine optimalen Aufzeichnungseigenschaften erbringt.

Aufgabe der Erfindung ist es daher, ein Registriergerät der eingangs genannten Art — das insbesondere für tragbare Kardiographen Verwendung finden soll — zu schaffen, bei dem sich optimale Aufzeichnungseigenschaften auf der vom vorderen Teil des Trägerschlittens gebildeten Schreibkante ergeben. Gleichzeitig soll auch die Bedienung des Gerätes möglichst einfach sein, wobei speziell der Austausch des Aufzeichnungsträgers für den Benutzer verbessert werden soll.

Die Aufgabe wird erfindungsgemäß dadurch gelöst, daß das Formteil an seinem zuerst eingesetzten vorderen Ende als Spreizhebel mit einer Auflagefläche ausgebildet ist, mittels welcher der Spreizhebel mit Einsetzen des separaten Trägerschlittens in das Gerätegehäuse eine weitere Andruckrolle, die in einem Vertikalschlitz des Gerätegehäuses gelagert ist, senkrecht nach oben verschiebt und in eine solche Betriebsstellung bringt, in der die Andruckrolle unterhalb der Schreibkante zu liegen kommt und in der der Abstand des Umfanges von Transportrolle und Andruckrolle geringer ist als der Umschlingungsdurchmesser des Aufzeichnungsträgers um die Schreibkante.

Beim Registriergerät nach der Erfindung greift der Spreizhebel erst bei eingesetztem Trägerschlitten in die relativ zueinander beweglichen Antriebs- sowie Andruck- und Umlenkrollen und bringt diese in die betriebsmäßige Position, derart, daß dadurch Umschlingungswinkel von mehr als 180° erreicht werden. Trotzdem ist kein kompliziertes Einfädeln des Aufzeichnungsträgers erforderlich.

Beim erfindungsgemäßen Registriergerät ist es also besonders vorteilhaft, daß durch die Spreizwirkung mittels des schreibkantenseitigen Formteiles des Trägerschlittens der Umschlingungswinkel des Aufzeichnungsträgers um die Schreibkante im Betriebszustand des Registriergerätes größer als 180° ist. Dadurch ist die Auflagefläche des Schreibzeigers auf dem Aufzeichnungsträger so weit wie möglich herabgesetzt; speziell bei Verwendung von thermosensitiven Aufzeichnungsträgern wird dadurch der Einbrennfleck des beheizten Schreibzeigers minimiert. Gleichzeitig wird durch die Hinterspannung eine besonders stramme Führung des Aufzeichnungsträgers erreicht. Die Auflösung

von schnell anfallenden, hochfrequenten Signalen auf dem thermosensitiven Aufzeichnungsträger wird dadurch auch insbesondere bei geringem Zeitvorschub des Aufzeichnungsträgers erheblich verbessert.

Daneben kann aufgrund der erfindungsgemäßen Konstruktion nun das Einsetzen des Trägerschlittens mit angelegten Aufzeichnungsträger in einfachster Weise mit nur einer Hand erfolgen. Ein Verrücken des Aufzeichnungsträgers nach Einsetzen des Schlittens ist praktisch unmöglich, da durch das Eingreifen des Spreizhebels der Aufzeichnungsträger sicher zwischen den einzelnen Rollen geführt wird. Nach optimal einfachem und sicherem Einlegen in eine Transportbahn ergibt sich nun also auch bei langsamen Vorschub optimale Führungssicherheit beim Transport des Aufzeichnungsträgers für den Registriervorgang.

In weiterer vorteilhafter Ausbildung der Erfindung weist das Gerätegehäuse für den Papierantrieb seitliche Führungsschlitze zum Einsetzen des Trägerschlittens auf, die derartig ausgebildet sind, daß der eingesetzte Trägerschlitten zeitlich nacheinander Translations- und Kipp- bzw. Schwenkbewegungen im Gerätegehäuse ausführen kann. Zur Arretierung des Trägerschlittens im Gerätegehäuse in Betriebsstellung weist das Gerätegehäuse eine gegen Federdruck vorgespannte Raste auf, die zur Entarretierung mittels Fingerdruck betätigbar ist. Durch einen in das Gerätegehäuse eingreifenden Federhebel wird nach Betätigung der Raste der gesamte Schlitten automatisch in eine Greifstellung für den Benutzer gebracht.

Der an sich komplizierte Bewegungsablauf einer Tauchbewegung beim Hineinbringen des Trägerschlittens in die Betriebsstellung ist also bei der Erfindung sehr vereinfacht. Wegen der nur noch durchzuführenden einfachen Elementarbewegungen (Translation, Kippen und umgekehrt) kann auch das Herausnehmen des Trägerschlittens vom Benutzer sehr einfach mittels Einhandbedienung durchgeführt werden. Die guten Bedienungseigenschaften sind insbesondere beim Einsatz des erfindungsgemäßen Registriergerätes für tragbare Elektrokardiographen, die im Ernstfall möglichst rasch betriebsbereit sein sollen, von Vorteil.

Einzelheiten der Erfindung ergeben sich aus der nachfolgenden Figurenbeschreibung anhand der Zeichnung. Es zeigt

Fig. 1 die Seitenansicht eines Gerätegehäuses mit Antrieb für den Aufzeichnungsträger in teilweise aufgebrochener Darstellung,

Fig. 2 eine Draufsicht auf das Gehäuse nach Fig. 1,

Fig. 3 die Seitenansicht eines Trägerschlittens für den Aufzeichnungsträger in teilweise aufgebrochener Darstellung,

Fig. 4 Gehäuse und Trägerschlitten nach Fig. 1 bis 3 in Wirkverbindung im Betriebszustand.

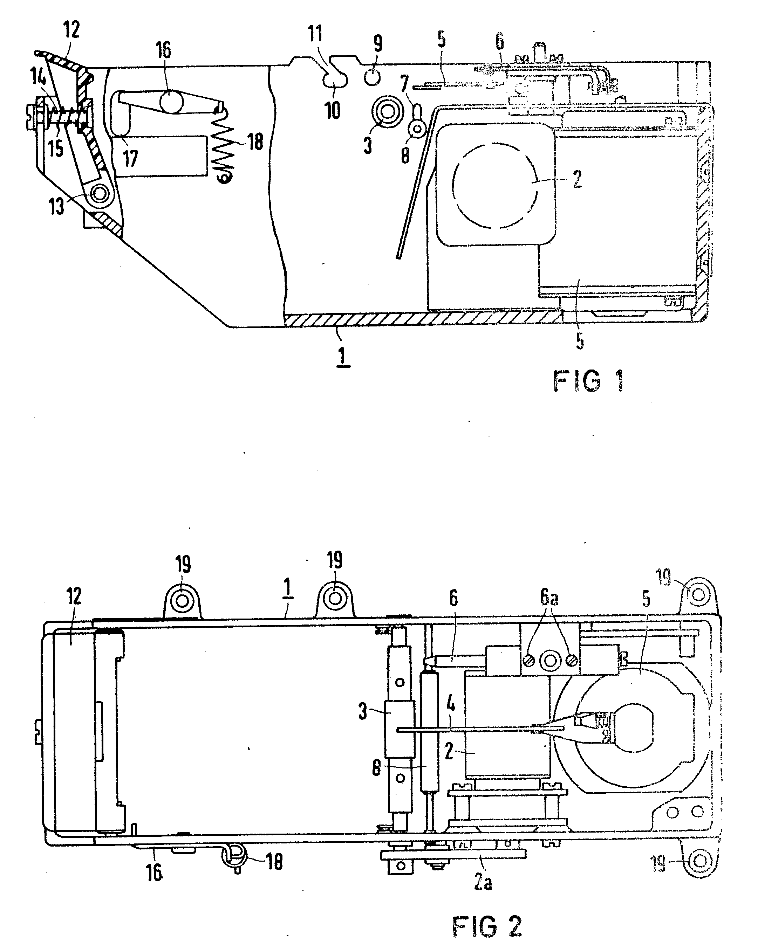

In den Fig. 1 und 2 ist das Gehäuse für den Papierantrieb mit 1 bezeichnet. Es ist in etwa als länglicher Kasten in den Maßen ca.

230 mm × 80 mm × 65 mm ausgebildet, wobei die Deckelfläche im Betriebszustand durch einen Registrierschlitten eingenommen wird. Die Höhe des Gehäuses 1 wird im wesentlichen durch den Durchmesser einer aufzunehmenden Vorratsrolle von Aufzeichnungsträgern, die Breite durch die verwendeten Normbreiten der Aufzeichnungsträger bestimmt. Es werden wahlweise die bei Einkanalregistrierern üblichen Normbreiten des Aufzeichnungsträgers von 50 oder 60 mm verwendet. Die linksseitige Ecke des kastenförmigen Gehäuses 1 ist aus Platzersparnis abgeschrägt.

In der Seitendarstellung nach Fig. 1 ist das Gehäuse 1 in beiden Endteilen im aufgeschnittenen Zustand, im mittleren Teil dagegen in Seitenansicht dargestellt, um die Funktion der erfindungswesentlichen Teile zu verdeutlichen: Im rechtsseitigen Teil des Gehäuses 1 ist ein Antriebsmotor 2 mit Getriebe für einen Aufzeichnungsträger angeordnet, der die Drehbewegung über ein Zahnradgetriebe 2a od. dgl. auf eine vorgelagerte Transportrolle 3 überträgt. Auf der Transportrolle 3 ist mittig eine elastische Gummirolle von 10 mm Durchmesser angeordnet. Oberhalb des Antriebsmotors 2 sind ein Schreibzeiger 4, welcher auf einer signalaufnehmenden, elektromagnetischen Spule 5 als Meßwerk befestigt ist, sowie ein weiterer handbetätigbarer Zeiger 6 zur Markierungsgabe auf dem Aufzeichnungsträger angeordnet. Dieser Zeiger 6 ist mittels Stellschrauben 6a so justierbar, daß die Markierungen am äußeren Rand des genormten Aufzeichnungsträgers aufgebracht werden können. Eine derartige Justiermöglichkeit ist bei Verwendung von Aufzeichnungsträgern unterschiedlicher Normbreiten von Vorteil. Auf diese Einheiten zur Registrierung wird in nachfolgender Beschreibung nicht weiter eingegangen. Dicht neben der Transportrolle 3 ist in einer vertikalen Ausnehmung 7 in der Seitenwand des Gehäuses 1 eine Andruckrolle 8 zur Auflage auf den Aufzeichnungsträger vertikal verschiebbar angeordnet. Die Funktion dieser Andruckrolle 8 als Andruckeinrichtung für einen Aufzeichnungsträger wird weiter unten noch im einzelnen beschrieben. Oberhalb der Transportrolle 3 sind weiterhin Lagernuten 9 für eine Abdeckung zum Schutz des Registrierteils vorhanden. Hinter den Lagernuten 9 sind im Gehäuse 1 auf beiden gegenüberliegenden Seiten horizontal verlaufende Ausnehmungen 10 angeordnet, welche von den oberen freien Kanten des Gehäuses 1 über schräg verlaufende Führungsschlitze 11 erreichbar sind. In diesem Bereich sind die Seitenwände des Gehäuses 1 zur Aufnahme der schrägen Schlitze 11 nach oben ausgelappt.

Am abgeschrägten (linksseitigen) Ende des Gehäuses 1 ist über die gesamte Gehäusebreite eine Raste 12 aus Kunststoff od. dgl. angebracht. Diese Raste 12 ist auf einer Drehwelle 13 gelagert und wird über einen im Gehäuse 1 gelagerten Bolzen 14 mit Druckfeder 15 vorgespannt. Der Raste 12 ist weiterhin in einer der seitlichen Wände des Gehäuses 1 ein schwenkbar gelagerter Federhebel 16 zugeordnet, welcher durch eine Ausnehmung 17 in der Gehäusewand mit einer abgekröpften Spitze in den Innenraum des Gehäuses 1 hineinragt, und der über eine Spiralfeder 18 ebenfalls vorgespannt ist. Das Gehäuse 1 weist weiterhin an seiner Grundfläche mehrere Laschen 19 auf, mit denen der gesamte Registrierteil auf einer Grundplatte befestigt wird.

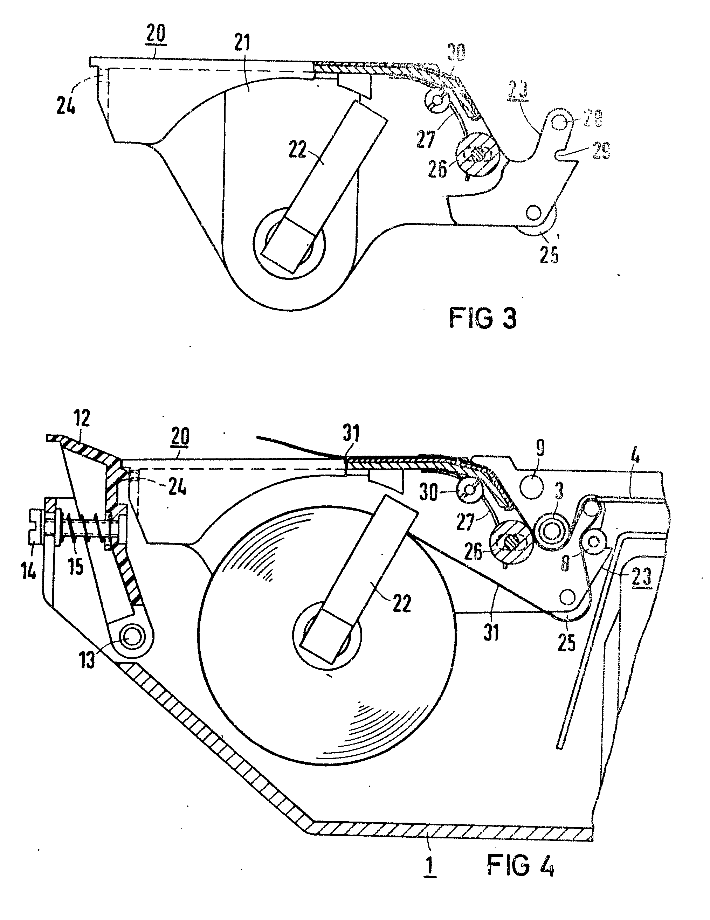

In der Fig. 3 ist der den Papierablauftisch bildende Teil des in das Gerätegehäuse 1 nach Fig. 1 und 2 einhängbaren Trägerschlittens mit 20 bezeichnet. An der Unterseite des Papierablauftisches 20 ist eine Papieraufhängevorrichtung 21 angeordnet, in der Vorratsrollen mit Aufzeichnungsträgern einhängbar sind. Der Aufzeichnungsträger wird dabei seitlich bündig durch eine Vorrichtung 22 nach dem deutschen Gebrauchsmuster 7 730 365 geführt. Der Trägerschlitten geht im rechtsseitigen Teil des Papierablauftisches in den eigentlichen Registriertisch über, wobei dieser abgekröpft vom Ablauftisch 20 als Vorformteil 23 ausgebildet ist. Am rückwärtigen Teil weist der Papierablauftisch 20 des Trägerschlittens eine innere Ausnehmung 24 auf, die mittels Raste 12 zur Arretierung des einhängbaren Trägerschlittens im Gerätegehäuse 1 gemäß Fig. 1 und 2 in der Betriebsstellung des Trägerschlittens dient. Das vordere abgekröpfte Formteil 23 des Trägerschlittens weist an der Unterkante eine starre Umlenkrolle 25 und eine drehbare Andruckrolle 26 mit Gummiumkleidung auf, zwischen denen am äußeren Ende des Formteils 23 eine dünne Welle 28 als Schreibkante für einen Aufzeichnungsträger angeordnet ist. Dabei ist die Andruckrolle 26 in einer Schlitzlagerung (nicht sichtbar, durchbrochen gezeichnet) verschiebbar gelagert. Zwei an den Außenkanten des Schlittens verlaufende Stahlfedern 27 drücken die Rolle 26 in der Schlitzlagerung nach vorn und wirken so als Federlager. Dicht unterhalb der Schreibkantenwelle 28 ist in dem abgekröpften Teil 23 eine in Betriebsstellung des Schlittens horizontal verlaufende Ausnehmung 29 eingebracht, die beim Eintauchen des Schlittens in das Gerätegehäuse 1 als sogenannte Mitnehmernut für die vertikal verschiebbare Welle 8 wirkt und deren Funktion noch im einzelnen erklärt wird. Weiter sind an den gegenüberliegenden Seiten des Schlittens zwei Steckbolzen 30 angeordnet, die zur Lagerung des Trägerschlittens im Gehäuse 1 nach Fig. 1 dienen und mit denen der Trägerschlitten in die Führungsschlitze 11 einsetzbar ist.

In der Fig. 4 ist das Gehäuse 1 der Fig. 1 mit eingesetztem Trägerschlitten und horizontal arretiertem Papierablauftisch 20 nach Fig. 3 dargestellt. Der Trägerschlitten (in seiner Gesamtheit nachfolgend mit 20 bezeichnet) ist in dieser Figur mit einer Vorratsrolle eines Aufzeichnungsträgers 31 bestückt, wobei der Aufzeichnungsträger 31 von der Rolle her als dick ausgezeichnete Linie funktionsgemäß zwischen Transport-, Andruck- und Umlenkrollen gezeichnet ist. Der eigentliche Registrierteil ist in dieser Figur weggelassen.

Das in den Fig. 1 bis 3 im einzelnen beschriebene Gerätegehäuse mit Transportantrieb und der darin einhängbaren Trägerschlitten haben in ihrem Zusammenwirken folgende Funktion: Nach Aufsetzen einer Vorratsrolle mit dem Aufzeichnungsträger 31 in die Aufhängevorrichtung 21 des Trägerschlittens 20 kann das vordere Ende des Aufzeichnungsträgers 31 über die Rollen 25, 28 und 26 gezogen und auf dem Papierablauftisch 20 festgehalten werden. Die Spur des Aufzeichnungsträgers 31 wird durch die Form des Vorformteils 23 in seinen wesentlichen Konturen vorgegeben. Mit einer Hand wird der gesamte Trägerschlitten gehalten und nun mittels Bolzen 30 in die schrägen Führungsschlitze 11 des Gehäuses 1 eingeführt. Dabei hat der Trägerschlitten 20 eine Neigung gegenüber der horizontalen Oberkante des Gehäuses 1 von etwa 45°, wobei in dieser Stellung durch diese Neigung das vordere, abgekröpfte Formteil 23 des Trägerschlittens 20 unter die Transportrolle 3 im Gehäuse 1 hinwegtauchen kann. Nachdem der Trägerschlitten 20 mittels Bolzen 30 bis zum Anschlag in den schrägen Führungsschlitzen 11 vorgeschoben ist, wird der Trägerschlitten 20 um die Bolzenachse 30 in Horizontalrichtung geschwenkt, so daß die Antriebswelle 3 zwischen den beiden Rollen 25 und 26 des Trägerschlittens 20 zu liegen kommt. Beim anschließenden Hochschwenken des vorderen abgekröpften Formteils 23 des Trägerschlittens 20 wird nun durch die Mitnehmernut 29 des Formteils 23 die vertikal beweglich gelagerte Andruckrolle 8 in der Ausnehmung 7 des Gehäuses 1 hochgeschoben. Die Antriebswelle 3 drückt dabei gleichzeitig die Gegendruckrolle 26 im Trägerschlitten 20 gegen Federdruck in der Schlitzlagerung nach hinten, so daß Transportrolle 3 und Gegendruckrolle 26 mit Gummiumkleidung mit dazwischen geführtem Aufzeichnungsträger 31 stramm aufeinander aufliegen. Wenn der Papierablauftisch 20 fast horizontal auf der Oberkante des Gehäuses 1 zu liegen kommt, ist er noch in der horizontalen Ausnehmung 10 des Gehäuses 1 in Horizontalrichtung verschiebbar, so daß sich ein exakter Sitz der Rolle 8 in der Mitnehmernut 29 einstellen kann. Der Papierablauftisch des Trägerschlittens 20 wird dabei gegen den Druck des Federhebels 16 bewegt, bis die unter Vorspannung stehende Raste 12 in die innere Ausnehmung 24 einrastet. Der gesamte Trägerschlitten gemäß Fig. 3 ist dann starr in horizontaler Lage im Gehäuse 1 arretiert. Zur Entarretierung des Trägerschlittens 20 braucht nun lediglich durch Fingerdruck die Raste 12 betätigt zu werden. Durch die Vorspannung des Hebels 16 über die Feder 18 wird der Trägerschlitten 20 dann nach oben gekippt; der gesamte Trägerschlitten 20 kann nun durch einen sinngemäß entgegengesetzten Bewegungsablauf aus dem Gerätegehäuse 1 herausgenommen werden.

Durch die Anordnung der Andruckrolle 8 in der Mitnehmernut 29 ergeben sich eine Hinterspannung des Aufzeichnungsträgers 31, und dadurch ein Umschlingungswinkel des Aufzeichnungsträgers 31 um die Schreibkante 28 von mehr als 180°. Der Aufzeichnungsträger 31 wird deswegen und durch den Federdruck der Andruckrolle 26 beim Ablauf straff geführt; die etwaige Bildung von losen Schleifen des Aufzeichnungsträgers 31 wird wirksam vermieden, so daß bei Registrierung auf thermosensitivem Material der Einbrennfleck des beheizten Schreibzeigers auf dem Aufzeichnungsträger 31 minimiert wird. Wegen der Größe des Einbrennflecks des beheizten Schreibzeigers 4 wird gerade bei geringem Zeitvorschub eines Aufzeichnungsträgers das Auflösungsvermögen bei der Registrierung ungünstig beeinflußt. Durch die Hinterspannung des Aufzeichnungsträgers 31 ist also gleichzeitig die Auflösung bei der Aufzeichnung schnell anfallender hochfrequenter Signale verbessert.

Für eine leichte Handhabung während des Betriebes des Registriergerätes ist es wichtig, daß die beweglichen Teile des Gerätegehäuses 1 mit Transportantrieb für den Aufzeichnungsträger 31 und Trägerschlitten 20 in ihren Maßen mit der Größe der wirksamen Federkräfte abgestimmt sind. Im beschriebenen Ausführungsbeispiel wird die Andruckrolle 26 mit einer Federkraft von 2mal 100 p belastet. Die Kraft der Schraubenfedern 13 und 18 zur Arretierung bzw. Entarretierung liegt etwa unterhalb des Gesamtwertes.

## Patentansprüche

1. Registriergerät zum Aufzeichnen elektrischer Signale auf einen bewegten bandförmigen Aufzeichnungsträger (31), mit einem Gerätegehäuse (1), das ein Antrieb mit Transportrolle (3) für den Aufzeichnungsträger (31) aufweist, und mit einem separaten, in das Gehäuse (1) einsetzbaren Trägerschlitten (20) als Träger eines Vorrates des Aufzeichnungsträgers (31), wobei der Trägerschlitten (20) eine Schreibkante (28) für wenigstens einen Schreibzeiger (4) aufweist und Trägerschlitten (20) und/oder Gerätegehäuse (1) Andruck- bzw. Umlenkrollen (8, 25, 26) für den Transport des Aufzeichnungsträgers (31) umfassen, von denen eine Andruckrolle (26) im Trägerschlitten (20) gegen den Druck einer Feder (27) verschiebbar gelagert ist, und wobei der Trägerschlitten (20) schreibkantenseitig ein Formteil (23) etwa in Form der im Betriebszustand gebildeten Spur des Aufzeichnungsträgers (31) bildet, längs dessen der Aufzeichnungsträger (31) lose anlegbar ist und das beim Einsetzen des Trägerschlittens (20) den Aufzeichnungsträger (31) mittels der Andruckrolle (26) entgegen der Wirkung der Feder (27) an die Transportrolle (3) andrückt, dadurch gekennzeichnet, daß das Formteil (23) an seinem zuerst eingesetzten vorderen Ende als Spreizhebel mit einer Auflagefläche (29) ausgebildet ist, mittels welcher der Spreizhebel mit Einsetzen des separaten Trägerschlittens (20) in das Gerätegehäuse (1) eine weitere Andruckrolle (8), die in einem Vertikalschlitz (7) des Gerätegehäuses (1) gelagert ist, senkrecht nach oben verschiebt und in eine solche Betriebsstellung bringt, in der die Andruckrolle (8) unterhalb der Schreibkante (28) zu liegen kommt und in der der Abstand des Umfanges von Transportrolle (3) und Andruckrolle (8) geringer ist als der Umschlingungsdurchmesser des Aufzeichnungsträgers (31) um die Schreibkante (28).

2. Registriergerät nach Anspruch 1, dadurch gekennzeichnet, daß das Formteil (23) zur Bildung der Auflagefläche eine in Betriebsstellung des Trägerschlittens (20) unterhalb der Schreibkante (28) horizontal verlaufende Aussparung (29) aufweist, die beim Einsetzen des Trägerschlittens (20) in das Gerätegehäuse (1) als Mitnehmernut für die vertikal verschiebbare Andruckrolle (8) wirkt.

3. Registriergerät nach Anspruch 1, dadurch gekennzeichnet, daß die Andruckrolle (26) im Trägerschlitten (20) horizontal verschiebbar gelagert ist.

4. Registriergerät nach Anspruch 1, dadurch gekennzeichnet, daß die Transportrolle (3) aus einer mit einem Antriebsmotor (2) über ein Getriebe (2a) in Wirkverbindung stehenden starren Welle mit einer mittig aufliegenden elastischen Rolle besteht.

5. Registriergerät nach Anspruch 1, dadurch gekennzeichnet, daß das Gerätegehäuse (1) zum Einsetzen des Trägerschlittens (20) seitliche Führungsschlitze (10, 11) aufweist, in denen der Trägerschlitten (20) mittels Bolzen so führbar ist, daß er erst eine Translations- und dann eine Schwenkbewegung ausführen kann.

6. Registriergerät nach Anspruch 5, dadurch gekennzeichnet, daß das Gerätegehäuse (1) im Bereich der Führungsschlitze (10, 11) nach oben vorstehende Auslappungen der Seitenwände aufweist.

7. Registriergerät nach Anspruch 1, dadurch gekennzeichnet, daß der Trägerschlitten (20) in seiner Betriebsstellung im Gerätegehäuse (1) durch eine gegen den Druck einer Feder (15) vorgespannte, durch Fingerdruck betätigbare Raste (12) arretiert ist.

8. Registriergerät nach Anspruch 7, dadurch gekennzeichnet, daß die Raste (12) über die gesamte Breite des Gerätegehäuses (1) verläuft.

9. Registriergerät nach einem der Ansprüche 1 bis 8, dadurch gekennzeichnet, daß ein in das Gerätegehäuse (1) eingreifender Federhebel (16) den entgegen der Wirkung der Hebelvorspannung in das Gerätegehäuse (1) eingesetzten Trägerschlitten (20) im entarretierten Zustand in eine Greifstellung für den Benutzer verschiebt.

## Claims

1. Recording device for recording electrical signals on a moving strip-like data carrier (31), comprising a device housing (1) which possesses a drive with a transport roller (3) for the data carrier (31), and comprising a separate carrier slide (20) which can be inserted into the housing (1) and which serves as carrier of a supply of the data carrier (31), wherein the carrier slide (20) possesses a recording edge (28) for at least one recording indicator (4), and the carrier slide (20) and/or the device housing (1) comprise pressure or guide rollers (8, 25, 26) for the transportation of the data carrier (31), of which one pressure roller (26) is mounted in the carrier slide (20) so as to be displaceable against the pressure of a spring (27), and wherein on the side of the recording edge the carrier slide (20) forms a moulded component (23) having approximately the shape of the track of the data carrier (31) which is formed in the operating state and along which the data carrier (31) can be applied in a slack condition and which, when the carrier slide (20) is inserted, presses the data carrier (31) against the transport roller (3) by means of the pressure roller (26) against the action of the spring (27), characterised in that at its front end which is the first to be inserted the moulded component (23) represents an expanding arm having a bearing surface (29) by means of which, when the separate carrier slide (20) is inserted into the device housing (1), the expanding arm displaces vertically upwards a further pressure roller (8) which is mounted in a vertical slot (7) of the device housing (1) and brings said further pressure roller into an operating position in which the pressure roller (8) comes to lie beneath the recording edge (28) and in which the distance between the periphery of the transport roller (3) and the pressure roller (8) is less than the loop diameter of the data carrier (31) around the recording edge (28).

2. Recording device as claimed in claim 1, characterised in that for the formation of the bearing surface the moulded component (23) possesses a recess (29) which runs horizontally beneath the recording edge (28) when the carrier slide (20) is in the operating position and which, when the carrier slide (20) is inserted into the device housing (1), acts as a keyway for the vertically displaceable pressure roller (8).

3. Recording device as claimed in claim 1, characterised in that the pressure roller (26) which is mounted in the carrier slide is displaced horizontally in the operating position of the carrier slide (20).

4. Recording device as claimed in claim 1, characterised in that the transport roller (3) consists of a rigid shaft which is actively connected to a drive motor (2) via a transmission (2a) with a centrally arranged elastic roller.

5. Recording device as claimed in claim 1, characterised in that for the insertion of the carrier slide (20) the device housing (1) possesses lateral guide slots (10, 11) in which the carrier slide (20) may be conducted by means of bolts in such manner that it can first execute a translational movement and then a pivoting movement.

6. Recording device as claimed in claim 5, characterised in that in the region of the guide slots (10, 11) the device housing (1) possesses upwards projecting overlaps of the side walls.

7. Recording device as claimed in claim 1, characterised in that in its operating position the carrier sliede (20) is locked in the device housing (1) by means of a locking mechanism (12) which is biased against the pressure of a spring (15) and which can be actuated by finger pressure.

8. Recording device as claimed in claim 7, characterised in that the locking mechanism (12) runs along the entire width of the device housing (1).

9. Recording device as claimed in any one of the claims 1 to 8, characterised in that a spring lever (16) which engages into the device housing (1) displaces, in the unlocked state, the carrier slide (20) which has been inserted into the device housing (1) against the action of the lever bias, into an engaging position for the user.

0 002 255

**Revendications**

1. Appareil enregistreur pour enregistrer des signaux électriques sur un support d'enregistrement (31) déplacé et en forme de bande, comportant un carter (1) pourvu d'un système d'entraînement à galet de transport (3) pour le support d'enregistrement (31) et un chariot-support (20) susceptible d'être monté dans le carter (1) et constituant un support pour une réserve du support d'enregistrement (31), ledit chariot-support (20) comportant un bord d'écriture (28) pour au moins une aiguille traçante (4) et le chariot-support (20) et/ou le carter (1) comportant des galets de pression ou galets de renvoi (8, 25, 26) pour le transport du support d'enregistrement, galets parmi lesquels un galet de pression (26) est monté dans le chariot-support (20) de manière à pouvoir être d+placé contre l'action d'un ressort (27), ledit chariot-support (20) formant du côté du bord d'écriture une pièce conformée (23) sensiblement de la forme de la piste tracée lors du fonctionnement par le support d'enregistrement (31) et le long de laquelle ce dernier peut être appliqué librement, alors que lors de la mise en place du chariot-support (20), ladite pièce conformée applique le support d'enregistrement (31), à l'aide du galet de pression (26) et à l'encontre de l'action d'un ressort (27), contre le galet de transport, caractérisé par le fait que l'extrémité antérieure de la pièce conformée (23) et mise en place en premier, est réalisée sous la forme d'un levier d'écartement à surface d'appui (29) à l'aide de laquelle, lors de la mise en place du chariot-support (20) dans le carter (1), ledit levier déplace verticalement vers le haut un autre galet de pression (8) monté dans une fente verticale (7) du carter et l'amène dans une position de fonctionnement dans laquelle le galet de pression (8) vient se situer sous le bord d'écriture (28) et dans laquelle la distance de la périphérie du galet de transport (3) au galet de pression (8) est inférieure au diamètre de la boucle de contact que fait le support d'enregistrement (31) sur le bord d'écriture (28).

2. Appareil enregistreur selon la revendication 1, caractérisé par le fait que la pièce conformée (23) possède, pour former la surface d'appui, une encoche (28) s'étendant horizontalement sous le bord d'écriture (28) lorsque le chariot-support (20) est en position de travail, encoche (29) qui agit lors de la mise en place du chariot-support (20) dans le carter (1), comme une encoche d'entraînement pour le galet de pression (8) déplaçable verticalement.

3. Appareil enregistreur selon la revendication 1, caractérisé par le fait que le galet de pression (26) monté sur le chariot-support est déplacé horizontalement lorsque le chariot-support (20) est en position de travail.

4. Appareil enregistreur selon la revendication 1, caractérisé par le fait que le galet de transport (3) est constitué par un arbre rigide en liaison avec un moteur d'entraînement (2) par l'intermédiaire d'une transmission (2a) et par un rouleau élastique monté en position médiane.

5. Appareil enregistreur selon la revendication 1, caractérisé par le fait que le carter (1), comporte, pour la mise en place du chariot-support (20), des fentes de guidage latérales (10, 11) dans lesquelles ledit chariot-support (20) est guidé à l'aide de chevilles de manière à exécuter d'abord un mouvement de translation et ensuite de basculement.

6. Appareil enregistreur selon la revendication 5, caractérisé par le fait que le carter (1) présente, au niveau des fentes de guidage (10, 11), des appendices des parois latérales, saillant vers le haut.

7. Appareil enregistreur selon la revendication 1, caractérisé par le fait que le chariot-support (20) est susceptible d'être fixé dans sa position de fonctionnement dans le carter (1) à l'aide d'une touche à encliquetage (12) actionnée par la pression d'un doigt, à l'encontre de la pression d'un ressort (15) chargeant ladite touche.

8. Appareil enregistreur selon la revendication 7, caractérisé par le fait que la touche à encliquetage (12) s'étend sur toute la largeur du carter (1).

9. Appareil enregistreur selon l'une des revendications 1 à 8, caractérisé par le fait qu'un levier élastique (16) pénétrant dans le carter (1) déplace le chariot-support (20) débloqué, et qui a été précédemment introduit dans le carter (1) à l'encontre de l'action de la pression préalable du levier, dans une position lui permettant d'être saisi par l'utilisateur.

FIG 1

FIG 2

9

0 002255

FIG 3

FIG 4

11